(19) Europäisches Patentamt

European Patent Office

Office européen des brevets

(11) Veröffentlichungsnummer: **0 373 597 B1**

(12) **EUROPÄISCHE PATENTSCHRIFT**

(51) Int. Cl.⁵: **C12N 15/54**, C12N 9/10, C12N 1/21, C12P 13/00, C12P 41/00, //(C12N1/21, C12R1:19)

(45) Veröffentlichungstag der Patentschrift: **29.09.93**

(21) Anmeldenummer: **89122939.5**

(22) Anmeldetag: **12.12.89**

(54) **Gen und Genstruktur, codierend für eine Aminotransferase, und Mikroorganismen, die dieses Gen exprimieren.**

(30) Priorität: **15.12.88 DE 3842174**
**26.09.89 DE 3932015**

(43) Veröffentlichungstag der Anmeldung:
**20.06.90 Patentblatt 90/25**

(45) Bekanntmachung des Hinweises auf die Patenterteilung:
**29.09.93 Patentblatt 93/39**

(84) Benannte Vertragsstaaten:
**AT BE CH DE ES FR GB IT LI NL SE**

(56) Entgegenhaltungen:
**EP-A- 0 248 357**
**EP-A- 0 265 852**
**EP-A- 0 344 683**
**EP-A- 0 349 965**

(73) Patentinhaber: **HOECHST AKTIENGESELL-SCHAFT**

**D-65926 Frankturt(DE)**

(72) Erfinder: **Bartsch, Klaus, Dr.**
**Feldbergstrasse 67**
**D-6374 Steinbach(DE)**
Erfinder: **Schulz, Arno, Dr.**
**Stauffenstrasse 22**
**D-6234 Hattersheim am Main(DE)**
Erfinder: **Uhlmann, Eugen, Dr.**
**Zum Talblick 31**
**D-6246 Glashütten/Taunus(DE)**

**Beschreibung**

In der (nicht vorveröffentlichten) deutschen Offenlegungsschrift 38 18 851 (entsprechend der am 06.12.89 veröffentlichten EP-A2 0 344 683) wurde bereits eine Aminotransferase (Transaminase) vorgeschlagen, die aus E. coli DH-1 isoliert wurde.

Es wurde nun das Gen gefunden, das für diese neue Transaminase codiert. Erfindungsgemäß kann man dadurch das Enzym in größeren Mengen als nach dem früheren Vorschlag herstellen, aber auch die spezifischen Transaminierungsreaktionen mit einem erfindungsgemäß transformierten Mikroorganismus durchführen. Die Isolierung und Charakterisierung des Gens erlaubt somit sehr viel effektivere Transaminierungen, als sie mit dem isolierten Enzym entsprechend dem früheren Vorschlag möglich sind.

In der deutschen Offenlegungsschrift 38 18 581 ist das neue Enzym u. a. durch die Aminosäuresequenz des N-Terminus charakterisiert. Die ersten 30 dieser Aminosäuren sind im folgenden wiedergegeben:

```
        1             5              10                15
NH₂-MET ASN SER ASN LYS GLU LEU MET GLN ARG ARG SER GLN ALA ILE
     16            20               25              30
     PRO ARG GLY VAL GLY GLN ILE HIS PRO ILE PHE ALA ASP ARG ALA --
```

Im Bereich der Aminosäuren 4 bis 10 findet sich Methionin, für das nur ein Triplett codiert, sowie vier Aminosäuren, die nur durch zwei Tripletts codiert werden. Lediglich Leucin ist im genetischen Code sechsfach "degeneriert".

Diese Sequenz eignet sich somit besonders gut zur Konstruktion einer Sonde aus 20 Nucleotiden (20 mer):

```
        5 -AAC AAA GAA TTA ATG CAA CG-3
              T   G   G C G       G A
                      C
                      T
```

Diese Sonde wurde in an sich bekannter Weise nach dem Phosphoamidit-Verfahren synthetisiert. Weiterhin wurde ein 38 mer des nicht-codierenden Stranges für die Aminosäuren 15 bis 27 synthetisiert.

```
3-TAG GGC GCG CCG CAA CCG GTC TAG GTG GGC TAG AAG CG-5
       T           T       T           T
```

Auch dieses Oligonucleotid wurde nach der Phosphoamidit-Methode synthetisiert.

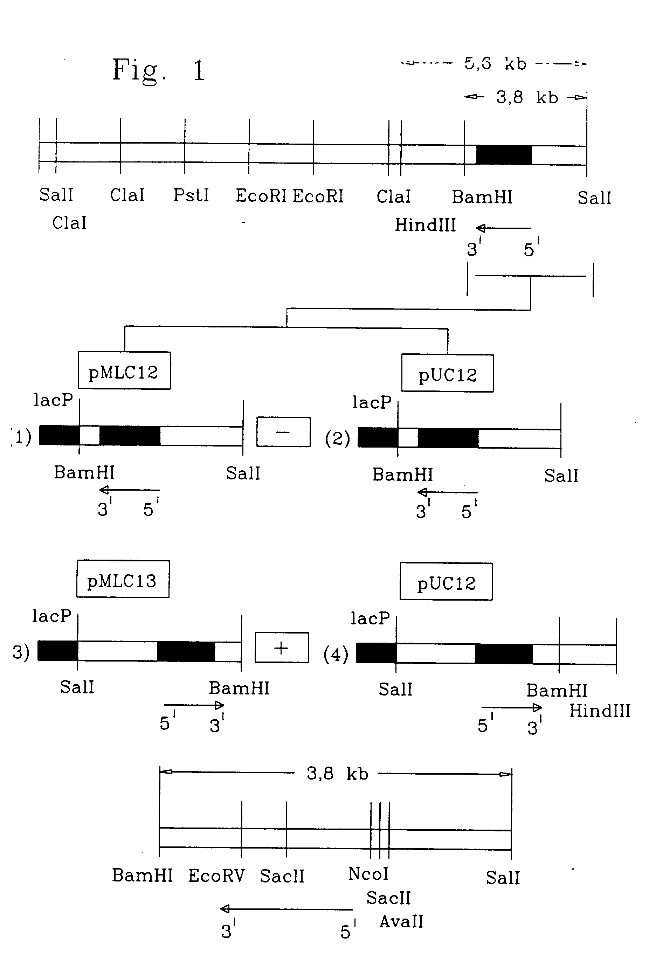

Diese Sonden wurden zum "Screening" einer Cosmid-Genbank von E. coli-DH 1 eingesetzt. Hybridisierungspositive Klone wurden zunächst auf erhöhte L-PPT-Transaminase-Aktivität getestet und dann durch Restriktionskartierung näher charakterisiert. Durch Subklonierung und Aktivitätstests von Teilfragmenten gelang es, die Lage des Gens im Genom einzugrenzen und anschließend durch Exonuclease-Abbau noch weiter zu definieren. So wurde zunächst ein 15 kb SalI-Fragment ermittelt, auf dem das erfindungsgemäße Gen liegt, und weiterhin ein 3,8 kb SalI/BamHI-Fragment, das die Feststellung der Orientierung des Gens ermöglichte (Fig. 1). Dieses Fragment enthält auch den eigenen Promotor des Gens. Durch Klonierung von Restriktionsfragmenten in geeignete Vektoren konnte so schon die Transaminase-Aktivität gegenüber dem Ausgangsstamm um das etwa Fünfzigfache erhöht werden.

Die Ausbeute an Enzym bzw. Enzymaktivität wird auch durch die Wahl geeigneter Anzuchtbedingungen beeinflußt. So spielt beispielsweise, abhängig von der Wahl des Expressionssystems, der Glucosegehalt im

EP 0 373 597 B1

Medium eine wesentliche Rolle: Bei Konzentrationen über 0,05 % kann sich ein drastischer Abfall der Enzymaktivität ergeben. Auch bei Kontrollstämmen, die nur die bakterienchromosomale Kopie des Transaminase-Gens exprimieren, ist diese Abhängigkeit nachweisbar.

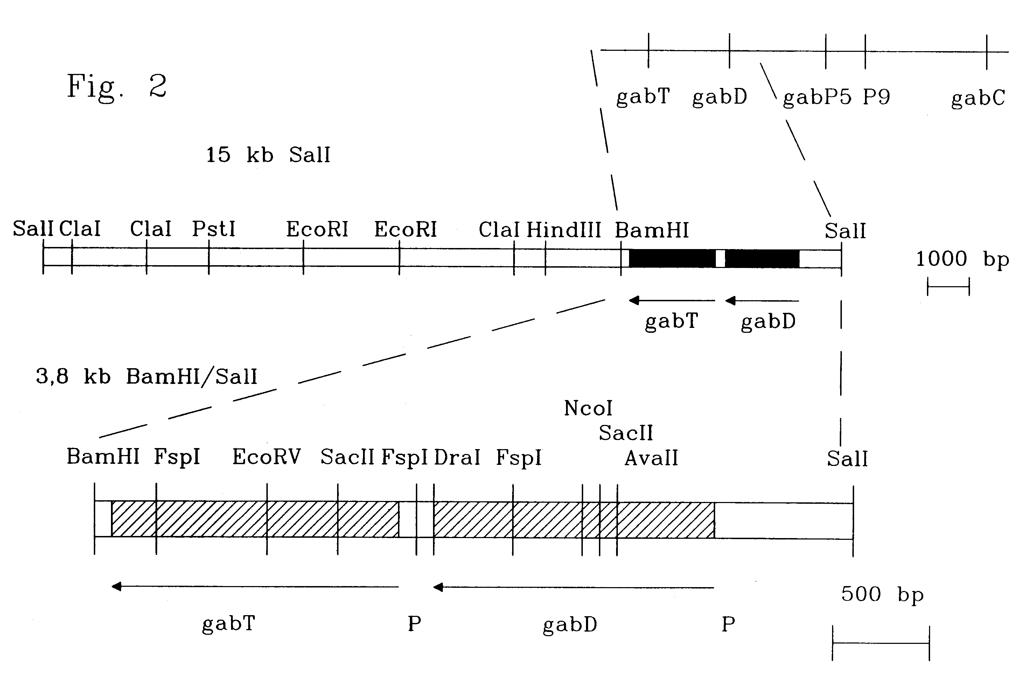

In Weiterentwicklung dieses Erfindungsgedankens konnte dann das für die Aminotransferase codierende Gen genauer lokalisiert werden: Das Gen befindet sich auf einem 1,6 kb Dral/BamHI-Fragment (Fig. 2), das einen offenen Leserahmen von 1281 Nukleotiden Länge (inklusive Stopp-Codon) enthält, der für ein Protein von 426 Aminosäuren codiert.

Die DNA-Sequenz ist in der Tabelle 1 wiedergegeben. Das ATG-Startcodon beginnt mit dem Nukleotid Nr. 275, das TAG-Stop-Codon beginnt mit dem Nukleotid Nr. 1553.

Die Tabelle 2 zeigt den codierenden Strang des Gens sowie die Aminosäuresequenz der erfindungsgemäßen Transaminase. Diese zeigt nur geringe Sequenzhomologien zu den übrigen bekannten Transaminasen aus E. coli (aspC, tyrB, hisC, ilvE, avtA und serC).

Auf Grund der Substratspezifität der L-PPT-Transaminase für 4-Aminobuttersäure (GABA) und eines Vergleichs der Restriktionskarte des 15 kb Sall-Fragmentes (siehe Fig. 1) mit der physikalischen Karte des E. coli K-12-Genoms [Kohara et al. (1987), Cell 50: 495-508] konnte das klonierte Transaminase-Gen als gabT, ein Locus aus dem E. coli K-12-gab-cluster bei 57,5 min. [Metzer et al. (1979), J. Bacteriol. 137; 1111-1118], identifiziert werden.

Die Kenntnis des Gens erlaubt es, das Strukturgen gezielt mit starken Promotoren zu versehen. Die so erhalten Genkonstrukte zeigen nicht nur höhere Expressionsraten als die vorher genannten Expressionsplasmide, sondern erlauben auch eine Steuerung ihrer Aktivität durch Induktoren. Weiterhin wird die Wahl von Expressionssystemen möglich, die keine Katabolitrepression aufweisen, wie das tac-System, so daß mit solchen Genkonstrukten transformierte Bakterien auch in Anwesenheit von Glukose im Nährmedium fermentiert werden können. Hierdurch werden hohe Zelldichten ermöglicht und damit hohe Ausbeuten, bezogen auf das Fermentervolumen, erreicht.

**Kurze Beschreibung der Figuren:**

Die Fig. 1 zeigt die Eingrenzung des erfindungsgemäßen Gens auf ein DNA-Fragment von 3,8 kb Größe.

Auf der Fig. 2 ist dargestellt, daß dieses Fragment neben dem gewünschten Gen (gab T) ein weiteres Gen (gab D) enthält.

Die Fig. 3 zeigt die zunächst entwickelten Vektoren pTrans1, pTrans2 und pTrans3.

Die Fig. 4 zeigt die Anordnung des erfindungsgemäßen Gens in den Vektoren pTrans4 bis pTrans7.

Die Fig. 5 zeigt die Anordnung des erfindungsgemäßen Gens in den Vektoren pTrans8 bis pTrans11.

Die Erfindung betrifft somit ein Gen für eine L-2-Amino-4-methylphosphinobuttersäure (L-PPT)-spezifische Transaminase, gelegen auf einem 1,6 kb Dral/BamHI-Fragment aus den Genom von E. coli-DH 1, mit der DNA-Sequenz gemäß Tabelle 2, und weiterhin ein Gen, codierend für ein Enzym mit der Aminosäuresequenz gemäß Tabelle 2 sowie für gleichwirkende Enzyme, deren Aminosäuresequenz sich von der gemäß Tabelle 2 durch Ergänzung, Weglassen oder Austausch von Aminosäuren ergibt.

Außerdem betrifft die Erfindung eine L-2-Amino-4-methylphosphinobuttersäure(L-PPT)-spezifische Transaminase mit einer Aminosäuresequenz, die sich von der gemäß Tabelle 2 durch Ergänzung, Weglassen oder Austausch von Aminosäuren ergibt.

Die Erfindung bezieht sich weiterhin auf Plasmide, enthaltend ein solches Gen, und auf Mikroorganismen, insbesondere E. coli, enthaltend ein derartiges Plasmid.

Die Erfindung betrifft auch ein Verfahren zur stereoselektiven Produktion von L-PPT aus (3-Carboxy-3-oxopropyl)-methylphosphinsäure durch Transaminierung mit Mikroorganismen, das dadurch gekennzeichnet ist, daß ein Mikroorganismus eingesetzt wird, der mit einem der vorstehend charakterisierten Plasmide transformiert ist oder wobei ein Enzym eingesetzt wird, das (wie vorstehend) durch Abwandlung der Aminosäuresequenz modifiziert ist.

In den folgenden Beispielen wird die Erfindung näher erläutert. Prozentangaben beziehen sich hierbei auf das Gewicht.

**Beispiel 1:**

Klonierung des L-PPT-Transaminase-Gens aus E. coli-DH 1/Konstruktion von Expressionsplasmiden

Chromosomale DNA aus E. coli-DH 1 wurde gemäß der in Ausubel et al. (1987), Current Protocols in Molecular Biology: 5.3.2.-5.4.3., 5.7.1.-5.7.3. beschriebenen Methode isoliert, partiell mit Sau3A gespalten

3

EP 0 373 597 B1

und im Agarose-Gel größenfraktioniert. DNA-Fragmente in der Größe von etwa 25-40 kb wurden in den mit BamHI geschnittenen Cosmid-Vektor pTBE [Grosveld, F. G. et al. (1982), Nucleic Acids Research 10: 6715 ] ligiert und in lambda-Phagen verpackt [Amersham: in vitro packaging system for Lambda DNA, Code No. 334Z, bzw. Maniatis et al. (1982), Molecular Cloning, A Laboratory Manual, Cold Spring Harbor: 296-299]. Nach Transfektion des Rezipientenstammes E. coli-DH 1 wurden 2000 Einzelklone isoliert, was mehreren Genomäquivalenten von E. coli entspricht. Zum Auffinden des gesuchten Gens in der angelegten Cosmid-Genbank wurden zwei zu Bereichen der N-terminalen Aminosäuresequenz des L-PPT-Transaminase-Proteins korrespondierende Oligonucleotide synthetisiert (20 mer und 38 mer, siehe Text).

Anschließend wurden die aus den Einzelklonen isolierten Cosmide (entsprechend Maniatis et al.: 331) mit Hilfe einer BRL "Dot-Blot"-Absaugapparatur an Nitrozellulosefilter (Schleicher und Schüll BA85) gebunden und mit den $^{32}$P-endmarkierten Oligonukleotiden hybridisiert (Ausubel et al.: 6.4.1.-6.4.4.). Von 4 hybridisierungspositiven Klonen zeigten 2 im L-PPT-Transaminase-Enzymtest (siehe unten, Beispiel 2) eine 3- bis 5-fach erhöhte Aktivität. Beide Klone enthielten eine identische 15 kb SalI-Insertion, deren Restriktionskarte in Fig. 1 dargestellt ist.

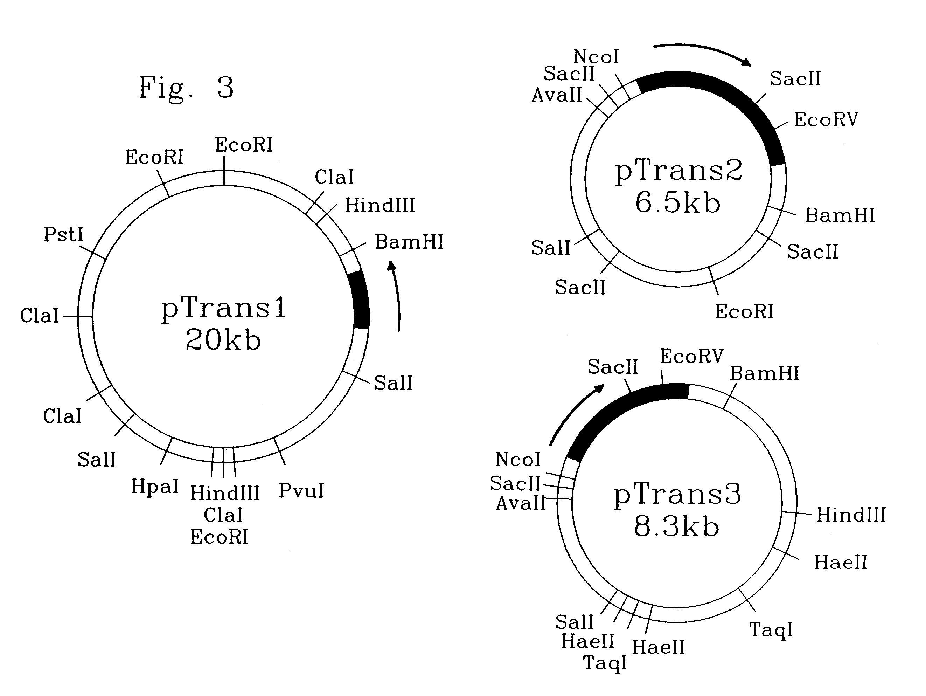

Von diesem DNA-Abschnitt wurden ein 5,6-kb HindIII/SalI- und ein 3,8 kb BamHI/SalI-Fragment, die beide mit den 5′-spezifischen Oligonukleotiden der L-PPT-Transaminase hybridisieren, in beiden Orientierungen hinter den E. coli-lac-Promoter in die Vektoren pUC12 und pMLC12/13 kloniert [Perbal, B. (1984), A Practical Guide to Molecular Cloning: 259-272] und die rekombinanten Plasmide anschließend auf Transaminase-Aktivität getestet (Fig. 1). Während die Enzymaktivitäten der Konstrukte (1) und (2) nur wenig über dem Hintergrund lagen, zeigten (3) und (4) (pTrans2 und pTrans3, Fig. 3) die das gleiche DNA-Fragment in der entgegengesetzten Orientierung zum lac-Promotor wie (1) und (2) enthielten, eine etwa 50-fach erhöhte L-PPT-Transaminase-Expression. Daraus ließ sich die Transkriptionsrichtung des Gens, wie in Fig. 1 dargestellt, ermitteln.

Die Lage des Transaminase-Gens auf dem 3,8 kb BamHI/SalI-Fragment wurde durch weitere Restriktionskartierung, sowie durch Herstellen einer Reihe von ExoIII/S1-Deletionen [Henikoff, S. (1984), Gene 28: 351-359] genauer festgelegt. Bei letzterem Verfahren wurde das in pMLC12/13 klonierte 3,8 kb Fragment jeweils von einem Ende her für verschieden lange Zeiten enzymatisch abgedaut. Die verkürzten Insertionen wurden anschließend auf Enzymaktivität getestet. Unter der Voraussetzung, daß bei nicht mehr aktiven DNA-Fragmenten Teile des Transaminase-Strukturgens deletiert waren, ließ sich die Lage des Gens, wie in Fig. 1 unten gezeigt, feststellen.

In Fig. 3 sind drei verschiedene rekombinante Plasmide dargestellt, die das klonierte L-PPT-Transaminase-Gen aus E. coli-DH 1 tragen und in den folgenden Beispielen verwendet werden.
Das Plasmid pTrans1 enthält das 15 kb SalI-Insert im Cosmid-Vektor pTBE und exprimiert die Transaminase unter Kontrolle des endogenen Promotors. Die beiden anderen Konstrukte sind Expressionsplasmide mit dem E. coli-lac-Promotor: pTrans2 enthält das 3,8 kb BamHI/SalI-Fragment in pMLC13, pTrans3 das 5,6 kb HindIII/SalI-Fragment in pUC12.

**Beispiel 2:**

L-PPT-Produktion in E. coli-DH 1 mit verschiedenen Transaminase-Expressionsplasmiden

E. coli-DH 1-Transformanten mit den rekombinanten Plasmiden pTrans1, pTrans2 und pTrans3 bzw. den Vektorplasmiden pTBE, pMLC13 und pUC12 als Kontrolle wurden in 10 ml Kulturen in LB-Medium [Luria-Bertani-Medium, Maniatis et al. (1982): 68] mit 50 $\mu$g des entsprechenden Antibiotikums (Ampicillin bei pTrans1, pTrans3, pTBE und pUC12 bzw. Chloramphenicol bei pTrans2 und pMLC13) für 15 h bei 37°C angezogen. Anschließend wurden die Zellen für 5 min. bei 5000 x g abzentrifugiert, 2 mal in je 5 ml 10 mM NaCl, 10 mM NaPhosphat, (pH = 7,5) gewaschen und für den Transaminase-Aktivitätstest in 1 ml "Reaktionsmix" (5 mM NaCl, 5 mM NaPhosphat, 30 g/l (3-Carboxy-3-oxopropyl)-methylphosphinsäure, 90 g/l L-Glutaminsäure, 100 mM Tris/HCl, pH = 8,0) resuspendiert. Die Zellen wurden 1 h bei 37°C unter Schütteln in dieser Lösung inkubiert und dann für 10 min. bei 95°C denaturiert. Die Reaktionsüberstände wurden im Aminosäurenanalysator (Biotronic Amino Acid Analyzer LC 5001, 3,2 x 130 mm BTC-2710 Säule) auf gebildetes L-PPT analysiert.

Die mit den verschiedenen Konstrukten erzielten Raum-Zeit-Ausbeuten sind in Tab. 3 zusammengefaßt. Die weitaus höchsten Enzymaktivitäten wurden mit den beiden lac-Expressionsplasmiden erreicht, wobei das pUC12-Derivat pTrans3, vermutlich wegen der größeren Kopienzahl pro Zelle, noch besser abschneidet als das pMLC13-Derivat pTrans2. Mit pTrans3 wurden Raum-Zeit-Ausbeuten gemessen, die etwa 60-fach über den Ergebnissen der mit pUC12-Vektorplasmid transformierten Kontrollzellen lagen.

4

Tabelle 3

| L-PPT-Produktion in E. coli-DH 1 mit verschiedenen Transaminase-Expressionsplasmiden | |
|---|---|
| **Plasmid:** | **Raum-Zeit-Ausbeute (mg L-PPT gebildet/l/h):** |
| pTBE | 100 |
| pMLC13 | 60 |
| pUC12 | 70 |
| pTrans1 | 300 |
| pTrans2 | 2400 |
| pTrans3 | 4300 |

**Beispiel 3:**

Einfluß der Glucosekonzentration im Anzuchtmedium auf die L-PPT-Transaminase-Aktivität

E. coli-DH 1-pTrans3 und E. coli-DH 1-pUC12 wurden in 10 ml LB-Medium ohne Glucose und mit steigenden Glucose-Konzentrationen (0,01 %, 0,05 %, 0,1 % und 0,5 %) wie in Beispiel 2 angezogen, aufgearbeitet und auf L-PPT-Transaminase-Aktivität getestet. Das Ergebnis zeigt die Tab. 4. Sowohl das lac-exprimierte Transaminase-Gen auf dem Plasmid pTrans3 als auch das chromosomale Gen aus dem Kontrollstamm (mit pUC12) wurden durch Glucosekonzentrationen > 0,05 % reprimiert. Die maximale L-PPT-Syntheserate konnte mit 0,05 % Glucose im Medium erzielt werden.

Tabelle 4

| Abhängigkeit der L-PPT-Transaminase-Aktivität in E. coli-DH-1 von der Glucosekonzentration im Anzuchtmedium | | |
|---|---|---|
| Glucosekonzentration im Medium (%) | Rel. Transaminase-Aktivität (%) | |
| | pUC12 | pTrans3 |
| 0 | 8 | 100[a] |
| 0,01 | 18 | 184 |
| 0,05 | 22 | 276 |
| 0,1 | 12 | 18 |
| 0,5 | 2 | 8 |

[a] Die Aktivität von pTrans3 ohne Glucose wurde gleich 100 % gesetzt.

**Beispiel 4:**

Überexpression des L-PPT-Transaminase-Proteins aus E. coli-DH1

E. coli-DH 1-pTrans3 und E. coli-DH 1-pUC12 wurden wie in Beispiel 3 angezogen. Die gewaschenen und in 1 ml 10 mM NaCl, 10 mM NaPhosphat (pH = 7,5) resuspendierten Zellen wurden durch 5 x 20 sec. Ultrabeschallung aufgeschlossen und Aliquots gleicher Proteinmengen dieser Rohextrakte auf ein 12,5 %iges SDS/Polyacrylamid-Gel aufgetragen [Laemmli, U. K. (1970), Nature 227: 680].

Im Proteinmuster der Extrakte aus E. coli-DH 1-pTrans3 erscheint das überexprimierte L-PPT-Transaminase-Protein als zusätzliche Bande von 43000 Dalton. Diese fehlt im Expressionsstamm in der Probe mit 0,5 % Glucose im Anzuchtmedium sowie im Kontrollstamm E. coli-DH 1-pUC12 mit 0,05 % Glucose.

**Beispiel 5:**

Sequenzierung des L-PPT-Transaminase-Gens aus E. coli K 12

Durch weitere Subklonierung und Aktivitätstests von Restriktionsfragmenten des 3,8 kb BamHI/SalI-Fragmentes konnte das L-PPT-Transaminase-Gen auf einem 1,6 kb DraI/BamHI-DNA-Stück lokalisiert werden (Fig. 2). Dieses wurde nach der Dideoxymethode [Sanger, F. et al. (1977), Proc. Natl. Acad. Sci. USA 74: 5463-5468] mit $\alpha$-[$^{35}$S]-dATP und Doppelstrang-DNA als "Templates" [Chen, E.Y. and Seeburg, P.H. (1985), DNA 4: 165-170] sequenziert. Dazu wurden durch ExoIII/S1-Nuklease-Verdauung hergestellte Deletionen, ausgehend vom 3'-Ende (BamHI-Schnittstelle) des Gens [Henikoff, S. (1984), Gene 28: 351-359], sowie eine Reihe von Restriktionsfragmenten des 1,6 kb DraI/BamHI-Stücks in bekannter Weise [Maniatis et al. (1982), Molecular Cloning, A Laboratory Manual, Cold Spring Harbor] in die Vektoren pMLC12/13 und pUC12/13 kloniert und mit handelsüblichen "Primern" (aus dem "pUC-Sequencing-Kit", Boehringer Mannheim, Best.-Nr. 1013 106) sequenziert. Außerdem wurden auch synthetische Oligonukleotide, die auf Grund schon vorhandener Sequenzinformationen hergestellt werden konnten (Phosphoamidit-Verfahren), als Sequenzierungsprimer verwendet. Die genaue Restriktionskarte des 1,6 kb DraI/BamHI-Fragmentes zeigt die Fig. 2.

**Beispiel 6:**

Herstellung von Expressionsplasmiden mit dem L-PPT-Transaminase-Strukturgen aus E. coli K-12

a) lac-Expressionsplasmide

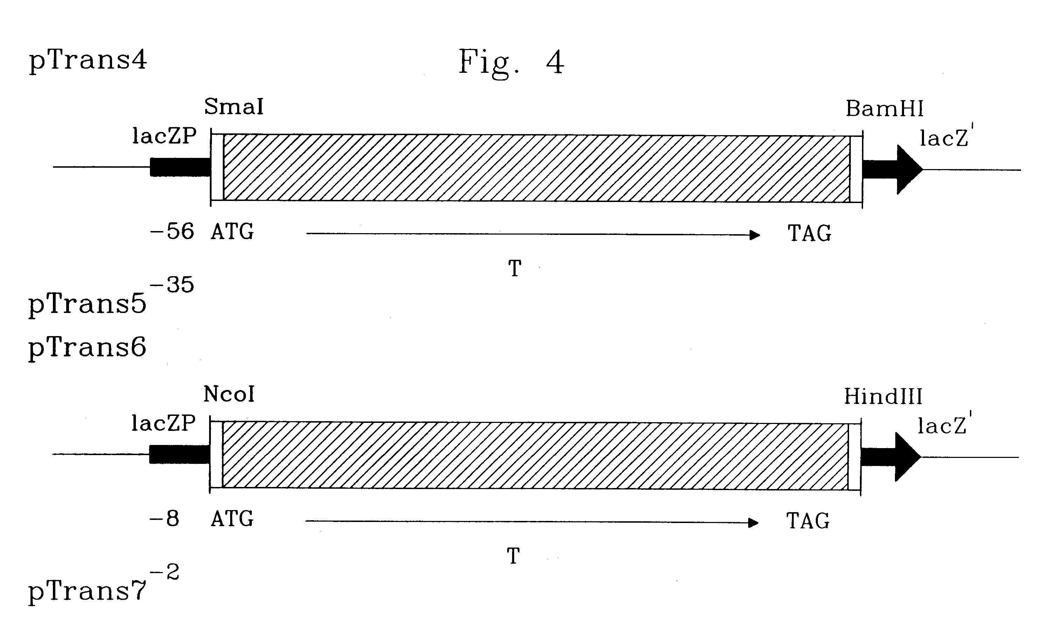

Um das Transaminase-Strukturgen mit einem anderen Promotor zu fusionieren, war es notwendig, den nichtcodierenden 5'-Bereich des 1,6 kb DraI/BamHI-Fragmentes oberhalb des Startcodons ATG weitestgehend zu deletieren. Zu diesem Zweck wurde das DNA-Fragment mit Hilfe der oben beschriebenen ExoIII/S1-Nuclease-Technik vom DraI-Ende her verkürzt. Zwei der so hergestellten Deletionen [-56 zum ATG und -35 zum ATG, siehe Tabelle 5, Konstrukte (I) und (II)] wurden als SmaI/BamHI-Fragmente hinter den lac-Promotor im Vektor pUC12, geschnitten mit SmaI/BamHI, kloniert. Die so erhaltenen Expressionsplasmide pTrans4 und pTrans5 sind in Fig. 4 dargestellt.

In einem anderen Ansatz wurde durch in-vitro-Mutagenese mit Hilfe der Taq-"Polymerase Chain Reaction"-Technik (PCR) [Higuchi et al. (1988), Nucleic Acids Research 16: 7351-7367] im Bereich des Startcodons ATG bzw. in der Position -6 eine NcoI-Schnittstelle in das Transaminase-Gen eingeführt [siehe Tabelle 5, Konstrukte (III) und (IV)]. Während die NcoI-Schnittstelle in der Position -6 das Transaminase-Strukturgen nicht beeinflußt, wird durch die Mutation im Bereich des Startcodons die Aminosäure 2 der Transaminase von Asn in Asp verändert. Dieser konservative Aminosäureaustausch hat jedoch keinen Einfluß auf die Aktivität des Enzymproteins. Auf Grund der eingeführten Restriktionsschnittstellen in den beiden Konstrukten (III) und (IV) konnte das Transaminase-Strukturgen nun ohne 5'-nichtcodierende Sequenzen jeweils als NcoI/HindIII-Fragment hinter den lac-Promotor in den Vektor pMG12 (pUC12-Derivat mit verändertem Polylinker: EcoRI-SmaI-BamHI-NcoI-NheI-HgiAI-PstI-KpnI-XbaI-ClaI-SalI-SacII-SphI-PvuI-HindIII), geschnitten mit NcoI/HindIII, kloniert werden (Fig. 4: Plasmide pTrans6 und pTrans7).

Um die Transaminase-Expression mit den verschiedenen Genkonstrukten zu überprüfen, wurden E. coli-JM103-Transformanten mit den rekombinaten Plasmiden pTrans4, pTrans5, pTrans6, pTrans7, sowie pTrans3 (siehe Beispiel 1) bzw. dem Vektorplasmid pUC12 als Kontrolle in 10 ml LB-Medium ohne und mit Glukose (0,5%) angezogen. Die L-PPT-spezifischen Transaminase-Aktivitäten wurden, wie in Beispiel 2 beschrieben, gemessen und in nmol L-PPT/min./mg Zellen angegeben. Die Ergebnisse sind in der Tabelle 6 zusammengefaßt. Die Enzymaktivitäten liegen mit diesen lac-Expressionsplasmiden etwa um den Faktor 2 höher als mit dem Plasmid pTrans3. Alle Konstrukte zeigen Katabolit-Repression in Anwesenheit von Glukose.

b) tac-Expressionsplasmide

Zur Expression des L-PPT-Transaminase-Gens mit dem hybriden tac-Promotor (lac- und trp-Anteile) wurde der Expressionsvektor pJF118u verwendet. Er ist ein Derivat von pKK223-3 und enthält direkt hinter der tac-Promotor-Sequenz einen Polylinker mit den Restriktionsschnittstellen EcoRI-SmaI-BamHI-SalI-PstI-HindIII. Außerdem exprimiert der Vektor das für den lac-Repressor codierende lacI-Gen, so daß die Aktivität des tac-Promotors durch IPTG induziert werden kann. Der tac-Promotor unterliegt keiner Katabolit-Repression durch Glukose.

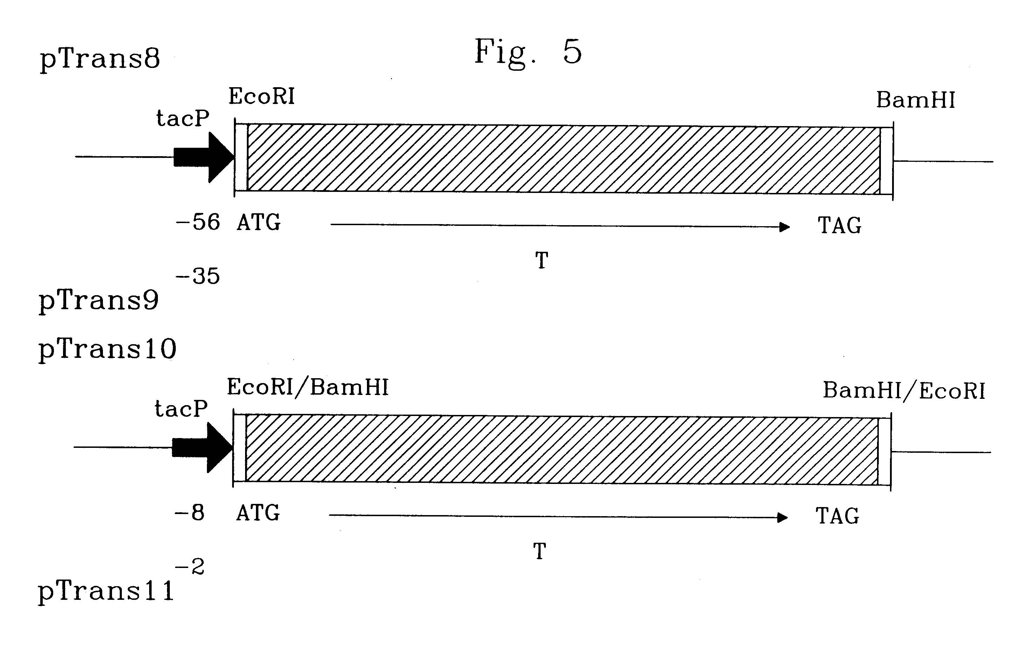

Die in Tabelle 5 dargestellten Transaminase-Genkonstrukte (I) und (II) (EXOIII/S1-Nuklease-Deletionen, -56 bzw. -35 zum ATG) wurden als EcoRI/BamHI-Fragmente hinter den tac-Promotor im Vektor pJF118u, geschnitten mit EcoRI/BamHI, kloniert. Die so erhaltenen rekombinanten Plasmide pTrans8 und pTrans9 zeigt die Fig. 5. Die durch in-vitro-Mutagenese hergestellten Transaminase-Genkonstrukte (III) und (IV) (siehe Tabelle 5) wurden als BamHI-Fragmente aus den Plasmiden pTrans6 und pTrans7 isoliert und die kohäsiven Enden mit Klenow-Enzym aufgefüllt. Diese Fragmente wurden hinter den tac-Promotor in pJF118u, geschnitten mit EcoRI und mit S1-Nuklease behandelt, kloniert. Von den isolierten Subklonen wurden nur solche weiterverwendet, die das L-PPT-Transaminase-Strukturgen in der richtigen Orientierung zum tac-Promotor enthielten (siehe Fig. 5, rekombinante Plasmide pTrans10 und pTrans11).

Zur Bestimmung der L-PPT-spezifischen Transaminase-Aktivitäten wurden E. coli-Jm103-Transformanten mit den rekombinanten Plasmiden pTrans8, pTrans9, pTrans10, pTrans11, sowie pTrans3 und dem Vektorplasmid pJF118u als Kontrolle in 10 ml LB-medium ohne und mit Glukose (0,5%) angezogen und nach 8h abgeerntet. In Parallelansätzen wurden die Zellen nach Erreichen einer $O.D._{600nm} = 0,5$ für 4 h mit 1 mM IPTG induziert und dann ebenfalls geerntet. Die Bestimmung der Transaminase-Aktivitäten erfolgte wie unter 6.a) beschrieben. Die Ergebnisse der Enzymmessungen sind in Tabelle 7 zusammengefaßt. Alle vier tac-Expressionsplasmide sind im Vergleich zum Plasmid pTrans3 durch IPTG induzierbar und zeigen keine Katabolit-Repression in Anwesenheit von Glukose. Die höchsten Enzymaktivitäten in Glukose-Medium wurden mit dem Plasmid pTrans11 erzielt und sind den mit den lac-Expressionskonstrukten in glukosefreiem Medium erreichten Werten vergleichbar.

**Beispiel 7:**

Produktion der L-PPT-Transaminase durch Fermentation

Für die Fermentation wurde das Transaminase-Expressionsplamsid pTrans7 in den Produktionsstamm E. coli W3110 [Campbell et al. (1978), Proc. Natl. Acad. Sci. USA 75: 2276-2280] transformiert. Die Zellen wurden in Fermentationsmedium [M9-Mineral-Medium gemäß Maniatis et al. (1982), Molecular Cloning, A Laboratory Manual, Cold Spring Harbor: 68-69, mit 4% Maltose als C-Quelle, 2% Casamino acids und 0,4% GABA] inokuliert und für 24 h bei 35°C in einem 10 l Fermenter (Biostat S, Braun Melsungen) mit konstanter Rührgeschwindigkeit (400 rpM), Belüftung (2 $m^3$/h) und automatischer pH-Regulierung (pH = 7,0) kultiviert. Die opTische Dichte der Kultur, sowie die L-PPT-spezifische Transaminase-Aktivität der Zellen wurden während der Fermentation durch Entnahme von Kontrollproben gemessen. Die Bakterien wurden nach 24 h bei einer spezifischen Transaminase-Aktivität von 0,35 nkat/mg Zellen (1 nkat = 1 nmol L-PPT/sec.) und einer optischen Dichte von 29 (entsprechend einer Zellmasse von 3 kg Feuchtgewicht) geerntet und anschließend mit dem Zell-Separator (Modell Westfalia, SA1-02-575) zehnfach konzentriert. Nach Zugabe von 20 mM Na-Phosphat, pH = 7,0, 0,01 mM Pyridoxalphosphat, 5 mM 2-MercapToethanol und 1 mM Phenylmethansulfonylfluorid (PMSF) wurden die Bakterien in einem "Micro Fluidizer" (Modell M-110 TIV, Micro Fluids, Newton USA) bei 800 bar aufgeschlossen. Der Rohextrakt wurde für 10 min. bei 70°C inkubiert und die Zelltrümmer sowie die ausgefallenen Proteine anschließend durch Zentrifugation für 20 min. bei 6000 xg abgetrennt. Nach dieser Behandlung enthielt der Überstand 16 g Protein mit einer L-PPT-spezifischen Transaminase-Aktivität von 7480 nkat/mg Protein. In auf gleiche Weise hergestellten Überständen des untransformierten Produktionsstammes W3110 konnte nur eine Transaminase-Aktivität von 200 nkat/mg Protein gemessen werden, was einer Steigerung der Enzymaktivität durch das rekombinante Plasmid pTrans7 auf das ca. 40-fache entspricht. Wie eine SDS-Gel-Analyse der Proteine [Laemmli, U.K. (1970), Nature 227: 680] zeigt, ist die L-PPT-Transaminase in den aufgearbeiteten Fermentationsüberständen nach der Hitzefällung bei 70°C das eindeutig dominierende Protein. Dieser Anreicherungsgrad der Transaminase ist ausreichend, um das Material direkt für die Trägerfixierung des Enzyms gemäß der in der deutschen Offenlegungsschrift 38 18 851 vorgeschlagenen Methode zu verwenden.

7

Tabelle 1

```
DraI                        FspI
TTTAAAGATGAAGCTGATGTGATTGCGCAAGCCAATGACACCGAGTTTGGCCTTGCCGCCTATTTCTACGCC
AAATTTCTACTTCGACTACACTAACGCGTTCGGTTACTGTGGCTCAAACCGGAACGGCGGATAAAGATGCGG
        12        24        36        48        60        72
--------------------------------------------------------------------------
CGTGATTTAAGCCGCGTCTTCCGGCGTGGGCGAAGCGCTGGAGTACGGCATCGTCGGCATCAATACCGGCATT
GCACTAAATTCGGCGCAGAAGGCGCACCCGCTTCGCGACCTCATGCCGTAGCAGCCGTAGTTATGGCCGTAA
        84        96        108       120       132       144
--------------------------------------------------------------------------
ATTTCCAATGAAGTGGCCCCCGTTCGGCGGCATCAAAGCCTCGGGTCTGGGTCGTGAAGGTTCGAAGTATGGC
TAAAGGTTACTTCACCGGGGCAAGCCGCCGTAGTTTCGGAGCCCAGACCCAGCACTTCCAAGCTTCATACCG
        156       168       180       192       204       216
--------------------------------------------------------------------------
ATCGAAGATTACTTAGAAATCAAATATATGTGCATCGGTCTTTAACTGGAGAATGCGAATGAACAGCAATAA
TAGCTTCTAATGAATCTTTAGTTTATATACACGTAGCCAGAAATTGACCTCTTACGCTTACTTGTCGTTATT
        228       240       252       264       276       288
--------------------------------------------------------------------------
AGAGTTAATGCAGCGCCGCAGTCAGGCGATTCCCCGTGGCGTTGGGCAAATTCACCCGATTTTCGCTGACCG
TCTCAATTACGTCGCGGCGTCAGTCCGCTAAGGGGCACCGCAACCCGTTTAAGTGGGCTAAAAGCGACTGGC
        300       312       324       336       348       360
--------------------------------------------------------------------------
CGCGGAAAACTGCCGGGTGTGGGACGTTGAAGGCCGTGAGTATCTTGATTTCGCGGGCGGGATTGCGGTGCT
GCGCCTTTTGACGGCCCACACCCTGCAACTTCCGGCACTCATAGAACTAAAGCGCCCGCCCTAACGCCACGA
        372       384       396       408       420       432
--------------------------------------------------------------------------
                                    SacII
CAATACCGGGCACCTGCATCCGAAGGTGGTGGCCGCGGTGGAAGCGCAGTTGAAAAAAACTGTCGCACACCTG
GTTATGGCCCGTGGACGTAGGCTTCCACCACCGGCGCCACCTTCGCGTCAACTTTTTTGACAGCGTGTGGAC
        444       456       468       480       492       504
--------------------------------------------------------------------------
CTTCCAGGTGCTGGCTTACGAGCCGTATCTGGAGCTGTGCGAGATTATGAATCAGAAGGTGCCGGGCGATTT
GAAGGTCCACGACCGAATGCTCGGCATAGACCTCGACACGCTCTAATACTTAGTCTTCCACGGCCCGCTAAA
        516       528       540       552       564       576
--------------------------------------------------------------------------
CGCCAAGAAAACGCTGCTGGTTACGACCCGGTTCCGAAGCGGTGGAAAACGCGGTAAAAATCGCCCGCGCCGC
GCGGTTCTTTTGCGACGACCAATGCTGGGCCAAGGCTTCGCCACCTTTTGCGCCATTTTTAGCGGGCGCGGCG
        588       600       612       624       636       648
--------------------------------------------------------------------------
CACCAAACGTAGCGGCACCATCGCTTTTAGCGGCGCGTATCACGGGCGCACGCATTACACGCTGGCGCTGAC
GTGGTTTGCATCGCCGTGGTAGCGAAAATCGCCGCGCATAGTGCCCGCGTGCGTAATGTGCGACCGCGACTG
        660       672       684       696       708       720
--------------------------------------------------------------------------
                                                        BssHII
CGGCAAGGTGAATCCGTACTCTGCGGGCATGGGGCTGATGCCGGGTCATGTTTATCGCGCGCTTTATCCTTG
GCCGTTCCACTTAGGCATGAGACGCCCGTACCCCGACTACGGCCCAGTACAAATAGCGCGCGCGAAATAGGAAC
        732       744       756       768       780       792
--------------------------------------------------------------------------
CCCGCTGCACGGCATAAGCGAGGATGACGCTATCGCCAGCATCCACCGGATCTTCAAAAAATGATGCCGCGCC
GGGCGACGTGCCCTATTCGCTCCTACTGCCGATAGCCGTCGTAGGTGGCCTAGAAGTTTTTACTACGGCGCGG
        804       816       828       840       852       864
--------------------------------------------------------------------------
```

Tabelle 1, Forts.

*EcoRV*
```
GGAAGATATCGCCGCCATCGTGATTGAGCCGGTTCAGGGCGAAGGCGGTTTCTACGCCTCGTCGCCAGCCTT
CCTTCTATAGCGGCGGTAGCACTAACTCGGCCAAGTCCCGCTTCCGCCAAAGATGCGGAGCAGCGGTCGGAA
      876         888         900         912         924         936
-----------------------------------------------------------------------------

  TATGCAGCGTTTACGCGCTCTGTGTGACGAGCACGGGATCATGCTGATTGCCGATGAAGTGCAGAGCGGCGC
  ATACGTCGCAAATGCGCGAGACACACTGCTCGTGCCCTAGTACGACTAACGGCTACTTCACGTCTCGCCGCG
      948         960         972         984         996        1008
-----------------------------------------------------------------------------
```

<div align="right">

Pvu I
ClaI
</div>

```
GGGGCGTACCGGCACGCTGTTTGCGATGGAGCAGATGGGCGTTGCGCCGGATCTTACCACCTTTGCGAAATC
CCCCGCATGGCCGTGCGACAGACGCTACCTCGTCTACCCGCAACGCGGCCTAGAATGGTGGAAACGCTTTAG
      1020        1032        1044        1056        1068        1080
-----------------------------------------------------------------------------
```

Pvul
ClaI
```
                                               BssHII
GATCGCGGGCGGCTTCCCGCTGGCGGGGCGTCACCGGGCGCGCGGAAGTAATGGATGCCGTCGCTCCAGGCGG
CTAGCGCCCGCCGAAGGGCGACCGCCCGCAGTGGCCCGCGCGCCTTCATTACCTACGGCAGCGAGGTCCGCC
      1092        1104        1116        1128        1140        1152
-----------------------------------------------------------------------------

  TCTGGGCGGCACCTATGCGGGTAACCCGATTGCCTGCGTGGCTGCGCTGGAAGTGTTGAAGGTGTTTGAGCA
  AGACCCGCCGTGGATACGCCCATTGGGCTAACGGACGCACCGACGCGACCTTCACAACTTCCACAAACTCGT
      1164        1176        1188        1200        1212        1224
-----------------------------------------------------------------------------

  GGAAAATCTGCTGCAAAAAGCCAACGATCTGGGGCAGAAGTTGAAAGACGGATTGCTGGCGATAGCCGAAAA
  CCTTTTAGACGACGTTTTTCGGTTGCTAGACCCCGTCTTCAACTTTCTGCCTAACGACCGCTATCGGCTTTT
      1236        1248        1260        1272        1284        1296
-----------------------------------------------------------------------------

  ACACCCGGAGATCGGCGACGTACGCGGGCTGGGGGGCGATGATCGCCATTGAGCTGTTTGAAGACGGCGATCA
  TGTGGGCCTCTAGCCGCTGCATGCGCCCGACCCCCGCTACTAGCGGTAACTCGACAAACTTCTGCCGCTAGT
      1308        1320        1332        1344        1356        1368
-----------------------------------------------------------------------------
```

```
                                               StuI
CAACAAGCCGGACGCCAAACTCACCGCCGAGATCGTGGCTCGCGCCCGCGATAAAGGCCTGATTCTTCTCTC
GTTGTTCGGCCTGCGGTTTGAGTGGCGGCTCTAGCACCGAGCGCGGGCGCTATTTCCGGACTAAGAAGAGAG
      1380        1392        1404        1416        1428        1440
-----------------------------------------------------------------------------
```

```
        FspI
CTGCGGCCCGTATTACAACGTGCTGCGCATCCTTGTACCGCTCACCATTGAAGACGCTCAGATCCGTCAGGG
GACGCCGGGCATAATGTTGCACGACGCGTAGGAACATGGCGAGTGGTAACTTCTGCGAGTCTAGGCAGTCCC
      1452        1464        1476        1488        1500        1512
-----------------------------------------------------------------------------

  TCTGGAGATCATCAGCCAGTGTTTTGATGAGGCGAAGCAGTAGCGCCGCTCCTATGCCGGAGAGCACTGCGC
  AGACCTCTAGTAGTCGGTCACAAAACTACTCCGCTTCGTCATCGCGGCGAGGATACGGCCTCTCGTGACGCG
      1524        1536        1548        1560        1572        1584
-----------------------------------------------------------------------------
```

```
        BamHI
GTCTTGTCCGGCCTACGGGGATCC
CAGAACAGGCCGGATGCCCCTAGG
      1596        1608
-----------------------------------------------------------------------------
```

**Tabelle 2**

```
                    289                    304                    319
ATG AAC AGC AAT AAA GAG TTA ATG CAG CGC CGC AGT CAG GCG ATT
Met Asn Ser Asn Lys Glu Leu Met Gln Arg Arg Ser Gln Ala Ile

                    334                    349                    364
CCC CGT GGC GTT GGG CAA ATT CAC CCG ATT TTC GCT GAC CGC GCG
Pro Arg Gly Val Gly Gln Ile His Pro Ile Phe Ala Asp Arg Ala

                    379                    394                    409
GAA AAC TGC CGG GTG TGG GAC GTT GAA GGC CGT GAG TAT CTT GAT
Glu Asn Cys Arg Val Trp Asp Val Glu Gly Arg Glu Tyr Leu Asp

                    424                    439                    454
TTC GCG GGC GGG ATT GCG GTG CTC AAT ACC GGG CAC CTG CAT CCG
Phe Ala Gly Gly Ile Ala Val Leu Asn Thr Gly His Leu His Pro

                    469                    484                    499
AAG GTG GTG GCC GCG GTG GAA GCG CAG TTG AAA AAA CTG TCG CAC
Lys Val Val Ala Ala Val Glu Ala Gln Leu Lys Lys Leu Ser His

                    514                    529                    544
ACC TGC TTC CAG GTG CTG GCT TAC GAG CCG TAT CTG GAG CTG TGC
Thr Cys Phe Gln Val Leu Ala Tyr Glu Pro Tyr Leu Glu Leu Cys

                    559                    574                    589
GAG ATT ATG AAT CAG AAG GTG CCG GGC GAT TTC GCC AAG AAA ACG
Glu Ile Met Asn Gln Lys Val Pro Gly Asp Phe Ala Lys Lys Thr

                    604                    619                    634
CTG CTG GTT ACG ACC GGT TCC GAA GCG GTG GAA AAC GCG GTA AAA
Leu Leu Val Thr Thr Gly Ser Glu Ala Val Glu Asn Ala Val Lys

                    649                    664                    679
ATC GCC CGC GCC GCC ACC AAA CGT AGC GGC ACC ATC GCT TTT AGC
Ile Ala Arg Ala Ala Thr Lys Arg Ser Gly Thr Ile Ala Phe Ser

                    694                    709                    724
GGC GCG TAT CAC GGG CGC ACG CAT TAC ACG CTG GCG CTG ACC GGC
Gly Ala Tyr His Gly Arg Thr His Tyr Thr Leu Ala Leu Thr Gly

                    739                    754                    769
AAG GTG AAT CCG TAC TCT GCG GGC ATG GGG CTG ATG CCG GGT CAT
Lys Val Asn Pro Tyr Ser Ala Gly Met Gly Leu Met Pro Gly His

                    784                    799                    814
GTT TAT CGC GCG CTT TAT CCT TGC CCG CTG CAC GGC ATA AGC GAG
Val Tyr Arg Ala Leu Tyr Pro Cys Pro Leu His Gly Ile Ser Glu

                    829                    844                    859
GAT GAC GCT ATC GCC AGC ATC CAC CGG ATC TTC AAA AAT GAT GCC
Asp Asp Ala Ile Ala Ser Ile His Arg Ile Phe Lys Asn Asp Ala

                    874                    889                    904
GCG CCG GAA GAT ATC GCC GCC ATC GTG ATT GAG CCG GTT CAG GGC
Ala Pro Glu Asp Ile Ala Ala Ile Val Ile Glu Pro Val Gln Gly
```

Tabelle 2, Forts.

```
            919                    934                    949
GAA GGC GGT TTC TAC GCC TCG TCG CCA GCC TTT ATG CAG CGT TTA
Glu Gly Gly Phe Tyr Ala Ser Ser Pro Ala Phe Met Gln Arg Leu

            964                    979                    994
CGC GCT CTG TGT GAC GAG CAC GGG ATC ATG CTG ATT GCC GAT GAA
Arg Ala Leu Cys Asp Glu His Gly Ile Met Leu Ile Ala Asp Glu

            1009                   1024                   1039
GTG CAG AGC GGC GCG GGG CGT ACC GGC ACG CTG TTT GCG ATG GAG
Val Gln Ser Gly Ala Gly Arg Thr Gly Thr Leu Phe Ala Met Glu

            1054                   1069                   1084
CAG ATG GGC GTT GCG CCG GAT CTT ACC ACC TTT GCG AAA TCG ATC
Gln Met Gly Val Ala Pro Asp Leu Thr Thr Phe Ala Lys Ser Ile

            1099                   1114                   1129
GCG GGC GGC TTC CCG CTG GCG GGC GTC ACC GGG CGC GCG GAA GTA
Ala Gly Gly Phe Pro Leu Ala Gly Val Thr Gly Arg Ala Glu Val

            1144                   1159                   1174
ATG GAT GCC GTC GCT CCA GGC GGT CTG GGC GGC ACC TAT GCG GGT
Met Asp Ala Val Ala Pro Gly Gly Leu Gly Gly Thr Tyr Ala Gly

            1189                   1204                   1219
AAC CCG ATT GCC TGC GTG GCT GCG CTG GAA GTG TTG AAG GTG TTT
Asn Pro Ile Ala Cys Val Ala Ala Leu Glu Val Leu Lys Val Phe

            1234                   1249                   1264
GAG CAG GAA AAT CTG CTG CAA AAA GCC AAC GAT CTG GGG CAG AAG
Glu Gln Glu Asn Leu Leu Gln Lys Ala Asn Asp Leu Gly Gln Lys

            1279                   1294                   1309
TTG AAA GAC GGA TTG CTG GCG ATA GCC GAA AAA CAC CCG GAG ATC
Leu Lys Asp Gly Leu Leu Ala Ile Ala Glu Lys His Pro Glu Ile

            1324                   1339                   1354
GGC GAC GTA CGC GGG CTG GGG GCG ATG ATC GCC ATT GAG CTG TTT
Gly Asp Val Arg Gly Leu Gly Ala Met Ile Ala Ile Glu Leu Phe

            1369                   1384                   1399
GAA GAC GGC GAT CAC AAC AAG CCG GAC GCC AAA CTC ACC GCC GAG
Glu Asp Gly Asp His Asn Lys Pro Asp Ala Lys Leu Thr Ala Glu

            1414                   1429                   1444
ATC GTG GCT CGC GCC CGC GAT AAA GGC CTG ATT CTT CTC TCC TGC
Ile Val Ala Arg Ala Arg Asp Lys Gly Leu Ile Leu Leu Ser Cys

            1459                   1474                   1489
GGC CCG TAT TAC AAC GTG CTG CGC ATC CTT GTA CCG CTC ACC ATT
Gly Pro Tyr Tyr Asn Val Leu Arg Ile Leu Val Pro Leu Thr Ile

            1504                   1519                   1534
GAA GAC GCT CAG ATC CGT CAG GGT CTG GAG ATC ATC AGC CAG TGT
Glu Asp Ala Gln Ile Arg Gln Gly Leu Glu Ile Ile Ser Gln Cys

            1549
TTT GAT GAG GCG AAG CAG TAG
Phe Asp Glu Ala Lys Gln ***
```

## Tabelle 5

**(I)**

5'                                                                                     3'

CGAAGATTACTTAGAAATCAAATATATGTGCATCGGTCTTTAACTGGAGAATGCGA ATG AAC AGC AAT ...

Met Asn  Ser Asn ...

**(II)**

5'                                                  3'

ATATATGTGCATCGGTCTTTAACTGGAGAATGCGA ATG AAC AGC AAT ...

Met Asn  Ser Asn ...

**(III)**

5'   Ncol                         3'

CCATGGGA ATG AAC AGC AAT ...

Met Asn  Ser Asn ...

**(IV)**

5'   Ncol                         3'

CC ATG GAC AGC AAT ...

Met Asp  Ser Asn ...

EP 0 373 597 B1

Tabelle 6: L-PPT spezifische Transaminase-Aktivitäten in Transformanten von E.coli JM103 mit verschiedenen lac-
Expressionsplasmiden.

| Plasmid: | Medium: | Spez. Transaminase-Aktivität [nmol L-PPT/min./mg Zellen]: |
|----------|---------|-----------------------------------------------------------|
| pTrans4  | LB            | 22,8 |
|          | LB + 0,5% Gluc. | 0,6 |
| pTrans5  | LB            | 25,7 |
|          | LB + 0,5% Gluc. | 1,2 |
| pTrans6  | LB            | 24,5 |
|          | LB + 0,5% Gluc. | 1,2 |
| pTrans7  | LB            | 24,9 |
|          | LB + 0,5% Gluc. | 0,9 |
| pTrans3  | LB            | 9,7 |
|          | LB + 0,5% Gluc. | 0,9 |
| pUC12    | LB            | 0,9 |
|          | LB + 0,5% Gluc. | 0,1 |

Tabelle 7: L-PPT spezifische Transaminase Aktivitäten in Transformanten von E.coli JM103 mit verschiedenen tac-
Expressionsplasmiden

| Plasmid: | Medium: | Spez. Transaminase-Aktivität [nmol L-PPT/min./mg Zellen]: |
|---|---|---|
| pTrans8 | LB | 11,4 |
| | LB + 1 mM IPTG | 20,5 |
| | LB + 0,5% Gluc. | 2,6 |
| | LB + 0,5% Gluc. + 1 mM IPTG | 12,9 |
| pTrans9 | LB | 9,6 |
| | LB + 1 mM IPTG | 21,7 |
| | LB + 0,5% Gluc. | 3,9 |
| | LB + 0,5% Gluc. + 1 mM IPTG | 11,3 |
| pTrans10 | LB | 2,3 |
| | LB + 1 mM IPTG | 16,6 |
| | LB + 0,5% Gluc. | 0,7 |
| | LB + 0,5% Gluc. + 1 mM IPTG | 4,5 |
| pTrans11 | LB | 5,9 |
| | LB + 1 mm IPTG | 20,9 |
| | LB + 0,5% Gluc. | 2,2 |
| | LB + 0,5% Gluc. + 1 mM IPTG | 22,1 |

14

## Tabelle 7: (Fortsetzung)

| Plasmid: | Medium: | Spez. Transaminase-Aktivität [nmol L-PPT/min./mg Zellen]: |
|---|---|---|
| pTrans3 | LB | 10,2 |
| | LB + 1 mM IPTG | 10,7 |
| | LB + 0,5% Gluc. | 0,3 |
| | LB + 0,5% Gluc. + 1 mM IPTG | 0,4 |
| pJF 118u | LB | 1,0 |
| | LB + 1 mM IPTG | 0,9 |
| | LB + 0,5% Gluc. | 0,2 |
| | LB + 0,5% Gluc. + 1 mM IPTG | 0,3 |

**Patentansprüche**

**Patentansprüche für folgende Vertragsstaaten : AT, BE, CH, DE, FR, GB, IT, LI, NL, SE**

1. Gen für eine L-2-Amino-4-methylphosphinobuttersäure (L-PPT)-spezifische Transaminase, gelegen auf einem 3,8 kb BamHI/SalI-Fragment, das aus dem Genom von E. coli-K 12 erhältlich ist.

2. Gen nach Anspruch 1, charakterisiert durch die Restriktionskarte gemäß Figur 1.

3. Gen für eine L-2-Amino-4-methylphosphinobuttersäure (L-PPT)-spezifische Transaminase, gelegen auf einem 1,6 kb DraI/BamHI-Fragment aus den Genom von E. coli-DH 1, mit der DNA-Sequenz gemäß Tabelle 2.

4. Gen, codierend für ein Enzym mit der Aminosäuresequenz gemäß Tabelle 2 sowie für gleichwirkende Enzyme, deren Aminosäuresequenz sich von der gemäß Tabelle 2 durch Ergänzung, Weglassen oder Austausch von Aminosäuren ergibt.

5. Plasmid, enthaltend ein Gen nach einem oder mehreren der Ansprüche 1 bis 4.

6. Mikroorganismus, enthaltend ein Plasmid nach Anspruch 5.

7. E. coli, enthaltend ein Plasmid nach Anspruch 5.

8. L-2-Amino-4-methylphosphinobuttersäure (L-PPT)-spezifische Transaminase mit einer Aminosäuresequenz, die sich von der gemäß Tabelle 2 durch Ergänzung, Weglassen oder Austausch von Aminosäuren ergibt.

9. Verfahren zur stereoselektiven Produktion von L-PPT aus (3-Carboxy-3-oxopropyl) methylphosphinsäure durch Transaminierung mit einem Mikroorganismus, dadurch gekennzeichnet, daß ein Mikroorganismus nach Anspruch 7 oder 8 eingesetzt wird.

10. Transaminierungverfahren, dadurch gekennzeichnet, daß ein Enzym nach Anspruch 8 eingesetzt wird.

**Patentansprüche für folgenden Vertragstaat : ES**

1.  Verfahren zur stereoselektiven Produktion von L-2-Amino-4-methylphosphinobuttersäure (L-PTT) aus (3-Carboxy-3-oxopropyl)-methylphosphinosäure und ihren Salzen durch Transaminierung mit Mikroorganismen, dadurch gekennzeichnet, daß man einen transformierten Mikroorganismus einsetzt, der eine Plasmid codierte α-PTT-spezifische Transaminase exprimiert, deren Gen auf einem 3,8 kb BamHI/SalI-Fragment liegt, das aus dem Genom von E. coli-K 12 erhältlich ist.

2.  Verfahren nach Anspruch 1, dadurch gekennzeichnet, daß das Gen auf einem 1,6 kb DraI/BamHI-Fragment liegt, das aus dem Genom von E. coli-DH1 erhältlich ist.

3.  Verfahren nach Anspruch 2, dadurch gekennzeichnet, daß das Gen eine Aminosäuresequenz gemäß Tabelle 2 codiert.

4.  Verfahren nach Anspruch 2, dadurch gekennzeichnet, daß das Gen ein Enzym codiert, dessen Aminosäuresequenz sich aus der gemäß Tabelle 2 durch Ergänzung, Weglassen oder Austausch von Aminosäuren ergibt.

5.  Verfahren nach Anspruch 3, dadurch gekennzeichnet, daß das Gen die Nucleotidsequenz gemäß Tabelle 2 hat.

6.  Transaminierungsverfahren, dadurch gekennzeichnet, daß ein Enzym eingesetzt wird, dessen Aminosäuresequenz sich aus der gemäß Tabelle 2 durch Ergänzung, Weglassen oder Austausch von Aminosäuren ergibt.

**Claims**

**Claims for the following Contracting States : AT, BE, CH, DE, FR, GB, IT, LI, NL, SE**

1.  A gene for a transaminase specific for L-2-amino-4-methylphosphinobutyric acid (L-PPT), located on a 3.8 kb BamHI/SalI fragment obtainable from the genome of E. coli K 12.

2.  A gene as claimed in claim 1, which has the restriction map shown in Figure 1.

3.  A gene for a transaminase specific for L-2-amino-4-methylphosphinobutyric acid (L-PPT), located on a 1.6 kb DraI/BamHI fragment from the genome of E. coli DH 1, having the DNA sequence shown in Table 2.

4.  A gene coding for an enzyme having the amino acid sequence shown in Table 2 as well as for enzymes which have the same action and whose amino acid sequence is derived from that shown in Table 2 by addition, deletion or exchange of amino acids.

5.  A plasmid containing a gene as claimed in one or more of claims 1 to 4.

6.  A microorganism containing a plasmid as claimed in claim 5.

7.  E. coli containing a plasmid as claimed in claim 5.

8.  A transaminase which is specific for L-2-amino-4-methylphosphinobutyric acid (L-PPT) and has an amino acid sequence which is derived from that shown in Table 2 by addition, deletion or exchange of amino acids.

9.  A process for the stereoselective production of L-PPT from (3-carboxy-3-oxopropyl)-methylphosphinic acid by transamination with a microorganism, which comprises employing a microorganism as claimed in claim 7 or 8.

10. A method of transamination which comprises employing an enzyme as claimed in claim 8.

**Claims for the following Contracting State : ES**

1. A process for the stereoselective production of L-2-amino-4-methylphosphinobutyric acid (L-PPT) from (3-carboxy-3-oxopropyl)-methylphosphinic acid by transamination with microorganisms, which comprises employing a transformed microorganism which expresses a plasmid-encoded transaminase specific for $\alpha$-PPT, whose gene is located on a 3.8 kb BamHI/SalI fragment obtainable from the genome of E. coli K 12.

2. The process as claimed in claim 1, wherein the gene is located on a 1.6 kb DraI/BamHI fragment obtainable from the genome of E. coli DH 1.

3. The process as claimed in claim 2, wherein the gene encodes an amino acid sequence shown in Table 2.

4. The process as claimed in claim 2, wherein the gene encodes an enzyme whose amino acid sequence is derived from that shown in Table 2 by addition, deletion or exchange of amino acids.

5. The process as claimed in claim 3, wherein the gene has the nucleotide sequence shown in Table 2.

6. A method of transamination which comprises employing an enzyme whose amino acid sequence is derived from that shown in Table 2 by addition, deletion or exchange of amino acids.

**Revendications**
**Revendications pour les Etats contractants suivants : AT, BE, CH, DE, FR, GB, IT, LI, NL, SE**

1. Gène d'une transaminase spécifique de l'acide L-2-amino-4-méthylphosphinobutyrique (L-PPT), situé sur un fragment BamHI/SalI de 3,8 kb, qui est obtenu à partir du genôme d'E. coli K 12.

2. Gène selon la revendication 1 caractérisé par la carte de restriction selon la figure 1.

3. Gène d'une transaminase spécifique de l'acide L-2-amino-4-méthylphosphinobutyrique (L-PPT), situé sur un fragment DraI/BamHI de 1,6 kb du génome d'E. coli-DH 1 comportant la séquence ADN selon le tableau 2.

4. Gène codant pour une enzyme contenant la séquence d'acides aminés selon le tableau 2 ainsi que pour des enzymes à effet identique, dont la séquence d'acides aminés résulte de celle montrée dans le tableau 2, par addition, délétion ou échange d'acides aminés.

5. Plasmide contenant un gène selon l'une quelconque des revendications 1 à 4.

6. Microorganisme, contenant un plasmide selon la revendication 5.

7. E. coli, contenant un plasmide selon la revendication 5.

8. Transaminase spécifique de l'acide L-2-amino-4-méthylphosphinobutyrique (L-PPT) avec une séquence d'acides aminés résultant de celle montrée dans le tableau 2, par addition, délétion ou échange d'acides aminés.

9. Procédé pour la production stéréosélective de L-PPT à partir d'acide (3-carboxy-3-oxopropyl)-méthylphosphinique par transamination avec un microorganisme caractérisé en ce qu'on utilise un microorganisme selon la revendication 7 ou 8.

10. Procédé de transamination caractérisé en ce qu'on utilise une enzyme selon la revendication 8.

**Revendications pour l'Etat contractant suivant : ES**

1. Procédé pour la production stéréosélective d'acide L-2-amino-4-méthylphosphinobutyrique (L-PPT) à partir d'acide (3-carboxy-3-oxopropyl)-méthylphosphinique et de ses sels par transamination avec des microorganismes caractérisé en ce qu'on utilise un microorganisme transformé exprimant une transaminase spécifique d'α-PTT codé par un plasmide, dont le gène est situé sur un fragment BamHI/SalI de 3,8 kb qui est obtenu à partir du genôme d'E. coli-K 12.

2. Procédé selon la revendication 1, caractérisé en ce que le gène est situé sur un fragment DraI/BamHI de 1,6 kb qui est obtenu à partir du genôme d'E. coli-DH 1.

3. Procédé selon la revendication 2 caractérisé en ce que le gène code pour une séquence d'acides aminés conforme au tableau 2.

4. Procédé selon la revendication 2, caractérisé en ce que le gène codant pour une enzyme, dont la séquence d'acides aminés est obtenue à partir de celle montrée dans le tableau 2, par addition, délétion ou échange d'acides aminés.

5. Procédé selon la revendication 3 caractérisé en ce que le gène contient la séquence de nucléotides selon le tableau 2.

6. Procédé de transamination caractérisé en ce qu'on utilise une enzyme dont la séquence d'acides aminés est obtenue à partir de celle montrée sur le tableau 2, par addition, délétion ou échange d'acides aminés.

# Fig. 1

Fig. 2

EP 0 373 597 B1

Fig. 3

pTrans4

Fig. 4

SmaI

lacZP

BamHI

lacZ'

−56 ATG

TAG

T

pTrans5 −35
pTrans6

NcoI

IacZP

HindIII

lacZ'

−8 ATG

TAG

T

pTrans7 −2

EP 0 373 597 B1

Fig. 5

pTrans8

EcoRI

BamHI

tacP

−56 ATG

TAG

T

−35

pTrans9

pTrans10

EcoRI/BamHI

BamHI/EcoRI

tacP

−8 ATG

TAG

T

−2

pTrans11

EP 0 373 597 B1